# EUROPÄISCHE PATENTSCHRIFT

(11) **EP 3 881 779 B1**
(45) Veröffentlichungstag und Bekanntmachung des Hinweises auf die Patenterteilung: **23.08.2023**
(21) Anmeldenummer: 20163365.8
(22) Anmeldetag: 16.03.2020
(51) Int. Cl.: A61B 17/14, A61B 17/15, A61B 17/17

(54) **VORRICHTUNG UND SYSTEM ZUR OPERATION EINES KNÖCHERNEN NASENGERÜSTS**
DEVICE AND SYSTEM FOR OPERATION OF AN OSSEOUS NASAL SUPPORT
DISPOSITIF ET SYSTÈME POUR L'OPÉRATION DU SQUELETTE OSSEUX DU NEZ

(43) Veröffentlichungstag der Anmeldung: 22.09.2021
(73) Patentinhaber: Bromba, Michael, 45529 Hattingen (DE)
(72) Erfinder: Bromba, Michael, 45529 Hattingen (DE)
(74) Vertreter: Betten & Resch

(56) Entgegenhaltungen:
- WO-A1-2019/166961
- WO-A2-91/13536
- US-A1- 2015 265 289

## Beschreibung

### TECHNISCHES GEBIET

Die Erfindung betrifft eine Vorrichtung und ein System zur Operation eines knöchernen Nasengerüsts.

### STAND DER TECHNIK

Formverändernde Nasenoperationen gehören weltweit zu den am häufigsten durchgeführten ästhetischen Eingriffen. Sie gehören allerdings auch zu den am schwierigsten erlernbaren Operationstechniken, die eine sehr lange Ausbildung der Operateure und eine entsprechende langjährige Erfahrung erfordern. Die nach dem heutigen Stand der Operationstechnik manuelle Durchführung einzelner entscheidender Operationsschritte bedingt eine sehr starke Streubreite der erzielten Ergebnisse und eine lange Lernkurve der Operateure mit entsprechenden Nachteilen für die Patienten. Bei Nasenoperationen (Rhinoplastik, Septorhinoplastik) zur plastischen Formgebung einer Nase werden die Nasenbeine entweder mit einem Meißel, einer Säge oder piezoelektrisch manuell durchtrennt. Wird bei der Gesichtsanalyse und Operationsplanung von einer Nasenoperation die Abtragung eines Nasenhöckers bzw. die Absenkung des Nasenrückens um z.B. 3 mm als die ästhetisch vorteilhafteste Veränderung ermittelt, mit dem Patienten besprochen und geplant, ist es während der Operation nach der heutigen Technik nicht möglich, diese Werte bzw. diese Form exakt auf das knöcherne Nasengerüst des Patienten zu übertragen und die Knochenschnitte so durchzuführen, dass das Operationsergebnis exakt und reproduzierbar mit den geplanten Werten bzw. der geplanten Form übereinstimmt. Die entsprechenden Instrumente zur Durchtrennung der Nasenbeine werden nach dem heutigen Stand der Technik von Hand von einem Operateur (zumeist dem betreuenden Arzt) geführt. Bei der Nasenoperation wird die Nasenhaut von dem knöchernen Nasengerüst gelöst und bleibt während der Nasenoperation im Bereich des Nasenrückens intakt. Bei einer sogenannten offenen Technik erfolgt ein Schnitt am Nasensteg und die Nase wird über diesen Schnitt freigelegt. Bei der sogenannten geschlossenen Technik werden keine äußeren Inzisionen durchgeführt. Sämtliche Operationsschritte erfolgen hier mit eingeschränkter Sicht auf das Operationsgebiet durch die Nasenlöcher. Bei beiden Techniken führt die manuelle Ausführung der Operationsschritte zu einer sehr großen Unsicherheit und Streubreite der tatsächlich durchgeführten Schnitte und zu einem erhöhten Risiko für unerwünschte Knochenbrüche (sogenannte "bad fractures"). Exakte und reproduzierbare Operationsergebnisse sind bei einer Nasenoperation nach der heutigen Technik und mit den im Stand der Technik verwendeten Operationswerkzeugen nicht möglich. Die Qualität der Operationsergebnisse hängt somit stark von der Fähigkeit des Operateurs ab und unterliegt daher Schwankungen. Dokument US 2015/265289 A1 betrifft ein chirurgisches Instrument, das zur Operation einer Hakennase verwendet wird, und insbesondere ein chirurgisches Instrument zur Entfernung eines Hakennasenhöckers durch eine Osteotomie,

Dokument WO 91/13536 A2 betrifft ein Instrument, das in der plastischen Chirurgie, insbesondere in der Rhinoplastik, verwendet wird.

Dokument WO 2019/166961 A1 betrifft eine Vorrichtung zur Führung eines chirurgischen Instruments.

Eine Aufgabe der Erfindung ist es, eine Vorrichtung und ein System für eine Operation eines knöchernen Nasengerüsts bereitzustellen, welche die Nachteile aus dem Stand der Technik weitestgehend abstellen, eine Unsicherheit und Streubreite der durch den Operateur durchgeführten Schnitte vermindern, ein Risiko für unerwünschte Knochenbrüche während der Operation minimieren und/oder exakte und reproduzierbare Operationsergebnisse von hoher Qualität ermöglichen, die weitgehend unabhängig von der Fähigkeit des Operateurs sind.

Diese Aufgabe wird durch eine Vorrichtung für eine Operation eines knöchernen Nasengerüsts nach Anspruch 1 und durch ein System für eine Operation eines knöchernen Nasengerüsts nach Anspruch 11 gelöst.

Die Vorrichtung für eine Operation eines knöchernen Nasengerüsts gemäß der vorliegenden Erfindung umfasst, unter anderem, ein Gestell, das eine im Wesentlichen umgekehrt u-förmige Form aufweist, während der Operation entlang einer Nasenrückenlängsachse ausgerichtet ist und dabei zumindest einen Bereich des knöchernen Nasengerüsts umgibt, wobei das Gestell eine erste und eine zweite Seite aufweist, die einander gegenüberliegend und während der Operation jeweils benachbart zu einer entsprechenden Seite des knöchernen Nasengerüsts angeordnet sind. Die Vorrichtung umfasst weiterhin ein an der ersten Seite des Gestells angeordnetes erstes Führungsmittel und ein an der zweiten Seite des Gestells angeordnetes zweites Führungsmittel, dergestalt, dass das erste und das zweite Führungsmittel ein nach innen in Richtung des knöchernen Nasengerüsts ausgerichtetes erstes Führungsmittelpaar bilden, wobei das erste und das zweite Führungsmittel zueinander parallel und im Wesentlichen parallel zur Nasenrückenlängsachse ausgerichtet sind und jeweils zumindest eine erste Führungskontaktfläche bereitstellen, und wobei die erste Führungskontaktfläche dazu eingerichtet ist, ein Operationswerkzeug zu einer zumindest bereichsweisen Durchtrennung des knöchernen Nasengerüsts in Richtung der Nasenrückenlängsachse translatorisch zu führen.

Die im Wesentlichen umgekehrt u-förmige Form des Gestells ist an die Form des Nasengerüsts und der umgebenden Nasenhaut angepasst und schützt die Nasenhaut vor Verletzungen z.B. durch das Operationswerkzeug. Dadurch wird das Auftreten von Komplikationen nach der Operation verringert. Das erste Führungsmittelpaar ermöglicht dem Operateur bei einer geplanten Nasenrücken-erhaltenden Operationstechnik eine exakte Abtragung des Nasenrückens als Knochendeckel, der am Ende der Operation zum Verschluss des entstandenen sogenannten "open roofs" ("offenen Dachs", d.h. des nach oben offenen Nasengerüst nach Entfernung des Nasenrückens) wieder aufgelegt, also replantiert und das Nasengerüst damit wieder geschlossen wird. Ferner ermöglicht die Bereitstellung des ersten Führungsmittelpaars eine Beschleunigung und Vereinfachung des gesamten Operationsverlaufs, da auf eine manuelle Durchführung der Schnitte verzichtet werden kann und der Knochendeckel zur Abdeckung des "open roofs" am Ende der Operation nicht mehr eingearbeitet werden muss. Die geplanten Schnitte erfolgen nicht mehr "blind", sondern geführt entlang einer durch das erste Führungsmittelpaarvorbestimmten Richtung, indem das Operationswerkzeug sicher im Führungsmittelpaar geführt wird. Ferner können dadurch operative Risiken wie zum Beispiel ungewollte Knochenabsplitterungen, fehlerhafte Schnitte, etc. reduziert werden. Durch den Einsatz der erfindungsgemäßen Vorrichtung kann aufgrund der sicheren, exakten, schnellen, detailliert planbaren und reproduzierbaren Durchführbarkeit der wesentlichen und ergebnisentscheidenden Operationsschritte die formverändernde Nasenoperation von auf diesem Gebiet tätigen Operateuren nach einer nur kurzen Einarbeitungszeit schonend für die Patienten, sicher und mit guten Ergebnissen umgesetzt werden. Damit wird die formverändernde Nasenoperation universell einsetzbar und für einen deutlich erweiterten Anwenderkreis mit einem erheblichen Kostenersparnispotential und einer verbesserten Patientensicherheit erschlossen. Die Vorrichtung ist sowohl bei einer offenen als auch bei einer geschlossenen Operationstechnik einsetzbar.

Gemäß einem Aspekt der vorliegenden Erfindung umfasst die Vorrichtung für eine Operation eines knöchernen Nasengerüsts ferner ein an der ersten Seite des Gestells angeordnetes drittes Führungsmittel und ein an der zweiten Seite des Gestells angeordnetes viertes Führungsmittel, dergestalt, dass das dritte und das vierte Führungsmittel ein nach innen in Richtung des knöchernen Nasengerüsts ausgerichtetes und ein zum ersten Führungsmittelpaar in der Höhe versetztes zweites Führungsmittelpaar bilden, wobei das dritte und vierte Führungsmittel zueinander parallel und im Wesentlichen parallel zur Nasenrückenlängsachse ausgerichtet sind und jeweils eine zweite Führungskontaktfläche bereitstellen, und wobei die zweite Führungskontaktfläche dazu eingerichtet ist, das Operationswerkzeug zu einer zumindest bereichsweisen Durchtrennung des knöchernen Nasengerüsts in Richtung der Nasenrückenlängsachse translatorisch zu führen.

Das zweite Führungsmittelpaar ermöglicht dem Operateur eine exakte Übertragung der geplanten Schnitte für die Veränderung der Form des Nasengerüsts und die exakte Absenkung der Profillinie des Nasenrückens. Ferner ermöglicht die Bereitstellung des zweiten Führungsmittelpaars eine Beschleunigung und Vereinfachung des gesamten Operationsverlaufs, da auf eine manuelle Durchführung der Schnitte verzichtet werden kann und der Knochendeckel zur Abdeckung des "open roofs" am Ende der Operation nicht mehr eingearbeitet werden muss. Die Vorrichtung muss dabei für die Durchführung von zumindest zwei Schnitten in einer unterschiedlichen Höhe nicht entnommen werden. Die geplanten Schnitte erfolgen nicht mehr "blind", sondern geführt entlang einer vorbestimmten Richtung des zweiten Führungsmittelpaars. Ferner können dadurch operative Risiken wie zum Beispiel ungewollte Knochenabsplitterungen, fehlerhafte Schnitte, etc. reduziert werden.

Gemäß einem weiteren Aspekt der vorliegenden Erfindung umfasst die Vorrichtung für eine Operation eines knöchernen Nasengerüsts ferner ein an einem unteren Endbereich der ersten Seite des Gestells angeordnetes fünftes Führungsmittel und ein an einem unteren Endbereich der zweiten Seite des Gestells angeordnetes sechstes Führungsmittel, wobei das fünfte und das sechste Führungsmittel jeweils in Richtung des knöchernen Nasengerüsts ausgerichtet sind und jeweils eine dritte Führungskontaktfläche bereitstellen, und wobei die dritte Führungskontaktfläche dazu eingerichtet ist, das Operationswerkzeug zu einer zumindest bereichsweisen Durchtrennung des knöchernen Nasengerüsts im Wesentlichen längs der Nasenrückenlängsachse translatorisch zu führen.

Das fünfte und sechste Führungsmittel ermöglichen dem Operateur eine exakte Durchführung der sogenannten lateralen Osteotomie, sofern diese erforderlich ist, für die Veränderung der Form des Nasengerüsts. Ferner ermöglicht die Bereitstellung des fünften und sechsten Führungsmittels eine Beschleunigung und Vereinfachung des gesamten Operationsverlaufs, da auf eine manuelle Durchführung der Schnitte verzichtet werden kann. Die Vorrichtung muss dabei für die Durchführung von zumindest zwei Schnitten in einer unterschiedlichen Höhe nicht entnommen werden. Die geplanten Schnitte erfolgen nicht mehr "blind", sondern geführt entlang einer vorbestimmten Richtung der fünften und sechsten Führungsmittel. Ferner können dadurch operative Risiken wie zum Beispiel ungewollte Knochenabsplitterungen, fehlerhafte Schnitte, etc. reduziert werden.

Gemäß einem weiteren Aspekt der vorliegenden Erfindung sind das erste Führungsmittelpaar und/oder das zweite Führungsmittelpaar und/oder das fünfte Führungsmittel und das sechste Führungsmittel im Wesentlichen senkrecht zu der Nasenrückenlängsachse in einer Höhe verstellbar.

Durch die Verstellbarkeit des ersten Führungsmittelpaars und/oder des zweiten Führungsmittelpaars und/oder des fünften Führungsmittels und des sechsten Führungsmittels in einer Höhe können die aus der Gesichtsanalyse und Operationsplanung vorbestimmten Werte bzw. die vorbestimmte Form exakt auf das knöcherne Nasengerüst übertragen und die Knochenschnitte können so durchgeführt werden, dass das Operationsergebnis exakt mit den ermittelten bzw. geplanten Werten bzw. der geplanten Form übereinstimmen. Ferner können durch die Verstellbarkeit des ersten Führungsmittelpaars und/oder des zweiten Führungsmittelpaars und/oder des fünften Führungsmittels und des sechsten Führungsmittels in einer Höhe mehrere verschiedenartige Schnitte in unterschiedlichen Höhen mit lediglich einer Vorrichtung durchgeführt werden. Die Vorrichtung kann daher mehrfach für eine Vielzahl von Operationen an verschiedenen Patienten angewendet werden. Dadurch wird die Flexibilität der Anwendung der Vorrichtung erhöht.

Gemäß einem Aspekt der vorliegenden Erfindung umfasst die Vorrichtung für eine Operation eines knöchernen Nasengerüsts ferner eine Kühlvorrichtung, die in dem oder benachbart zu dem Gestell angeordnet und dazu eingerichtet ist, zumindest einen zu dem oder den Führungsmitteln benachbarten Bereich der Vorrichtung zu kühlen.

Durch die Kühlvorrichtung kann im Betrieb das Nasengerüst effektiv und automatisiert gekühlt werden. Dadurch wird ein Absterben des Knochengewebes des Nasengerüsts aufgrund von einer Überhitzung des Gewebes verhindert. Ein entnommener und bearbeiteter Teil des Nasengerüsts, wie z.B. ein entnommener Teil des Nasenrückens, bleibt somit während der Operation intakt und kann, je nach gewählter Operationstechnik, replantiert werden. Die Wahrscheinlichkeit, dass der replantierte Teil des Nasengerüsts von dem Körper abgestoßen wird, wird dadurch verringert. Ferner kann durch die Kühlvorrichtung während der Operation das Operationswerkzeug effektiv und automatisiert gekühlt werden. Dadurch kann der Verschleiß des Operationswerkzeugs verringert werden.

Gemäß einem weiteren Aspekt der vorliegenden Erfindung umfasst die Vorrichtung für eine Operation eines knöchernen Nasengerüsts ferner zumindest ein Fixierungsmittel, das an der ersten oder zweiten Seite oder an einem die erste und die zweite Seite verbindenden dachförmigen Abschnitt des Gestells nach innen ausgerichtet angeordnet und dazu eingerichtet ist, das Gestell während der Operation relativ zu dem knöchernen Nasengerüst zu positionieren und daran zu fixieren.

Durch das zumindest eine Fixierungsmittel kann das Gestell in einer exakten Position relativ zu dem Nasengerüst angeordnet und fixiert werden. Dies ermöglicht die Durchführung von exakten Schnitten an dem Nasengerüst. Ferner kann dadurch verhindert werden, dass sich die Vorrichtung während der Durchführung der Schnitte an dem Nasengerüst ungewollt verschiebt.

Gemäß einem weiteren Aspekt der vorliegenden Erfindung umfasst die Vorrichtung für eine Operation eines knöchernen Nasengerüsts ferner zumindest drei Fixierungsmittel, dergestalt, dass jeweils ein Fixierungsmittel an der ersten und zweiten Seite des Gestells und ein Fixierungsmittel an dem dachförmigen Abschnitt des Gestells angeordnet sind.

Durch die Bereitstellung von zumindest drei Fixierungsmitteln kann das Gestell in einer exakten Position relativ zu dem Nasengerüst angeordnet und fixiert werden und die Belastung auf das Nasengerüst durch Kräfte, die über die Fixierungsmittel auf das Nasengerüst übertragen werden, kann reduziert werden. Dies ermöglicht die Durchführung von exakten Schnitten an dem Nasengerüst. Ferner kann dadurch verhindert werden, dass sich die Vorrichtung während der Durchführung der Schnitte an dem Nasengerüst ungewollt verschiebt.

Gemäß einem weiteren Aspekt der vorliegenden Erfindung umfasst das oder die Fixierungsmittel jeweils Schrauben oder Stifte zur Fixierung der Vorrichtung am knöchernen Nasengerüst.

Durch die Bereitstellung von Schrauben oder Stiften als Fixierungsmittel kann das Gestell einfach und exakt in einer vorbestimmten Position relativ zu dem Nasengerüst angeordnet und fixiert werden. Dadurch kann verhindert werden, dass sich die Vorrichtung während der Durchführung der Schnitte an dem Nasengerüst ungewollt verschiebt.

Gemäß einem weiteren Aspekt der vorliegenden Erfindung umfasst das Gestell weiterhin eine glatte und/oder beschichtete und/oder keimabweisende Oberfläche.

Durch eine glatte und/oder beschichtete und/oder keimabweisende Oberfläche des Gestells können im Betrieb Verletzungen der umgebenden Nasenhaut und des knöchernen Nasengerüsts verhindert werden. Ferner wird dadurch verhindert, dass sich Keime an dem Gestell ablagern, wodurch die Gefahr einer Infektion des Patienten verringert wird. Durch den atraumatischen Operationsablauf, den die Vorrichtung ermöglicht, wird der gesamte Heilungsprozess begünstigt und beschleunigt und das Auftreten von Komplikationen nach der Operation kann verringert werden. Außerdem wird dadurch ermöglicht, dass die Vorrichtung nach dem Gebrauch gereinigt, sterilisiert und für eine weitere Benutzung bei weiteren Operationen an anderen Patienten wiederverwendet werden kann, was zu einer Kostenreduktion für den Operateur führen kann. Ferner kann die Vorrichtung aus einem entsprechenden Material als Einmalinstrument gefertigt werden, welches dem Operateur als steriles Produkt zur Verfügung gestellt wird.

Gemäß einem weiteren Aspekt der vorliegenden Erfindung ist eine stirnseitig ausgerichtete Kante des Gestells abgerundet.

Durch die Bereitstellung einer abgerundeten stirnseitig ausgerichteten Kante des Gestells wird insbesondere bei der Einführung der Vorrichtung zwischen die Nasenhaut und das Nasengerüst verhindert, dass die Nasenhaut oder das Nasengerüst durch das Gestell verletzt wird. Dadurch wird das Auftreten von Komplikationen nach der Operation verringert.

Das System für eine Operation eines knöchernen Nasengerüsts gemäß der vorliegenden Erfindung umfasst die Vorrichtung zur Operation eines knöchernen Nasengerüsts, und das Operationswerkzeug zu einer zumindest bereichsweisen Durchtrennung des knöchernen Nasengerüsts im Wesentlichen längs der Nasenrückenlängsachse.

Das Operationswerkzeug ermöglicht die vorteilhafte Verwendung der Vorrichtung für eine Operation eines knöchernen Nasengerüsts.

Gemäß einem Aspekt der vorliegenden Erfindung ist das Operationswerkzeug ein Meißel, eine oszillierende Säge oder ein Piezohandstück.

Durch die Bereitstellung eines Meißels, einer oszillierenden Säge oder eines Piezohandstücks können die für die Operation eines knöchernen Nasengerüsts erforderlichen Schnitte vorteilhaft mit der Vorrichtung für die Operation eines knöchernen Nasengerüsts durchgeführt werden.

Gemäß einem weiteren Aspekt der vorliegenden Erfindung sind die Führungskontaktflächen der Führungsmittel derart ausgestaltet, dass sie zumindest eine Seite des Operationswerkzeugs in Richtung der Nasenrückenlängsachse umgeben und das Operationswerkzeug während der Operation translatorisch führen.

Durch die Bereitstellung der Führungskontaktflächen können die für die Operation eines knöchernen Nasengerüsts erforderlichen Schnitte mit einer hohen Genauigkeit durchgeführt werden.

Gemäß einem weiteren Aspekt der vorliegenden Erfindung umfasst das System für eine Operation eines knöchernen Nasengerüsts ferner eine Absaugvorrichtung, die an der Vorrichtung oder an dem Operationswerkzeug angeordnet ist und dazu eingerichtet ist, ein Kühlmedium während der Operation aus einer Umgebung des Gestells und des knöchernen Nasengerüsts abzusaugen.

Durch die Bereitstellung der Absaugvorrichtung kann das während der Operation verwendete Kühlmedium, das beispielsweise durch Blut oder Knochensplitter kontaminiert wurde, aus einem Bereich des knöchernen Nasengerüsts und des Gestells effektiv abgesaugt werden. Dies stellt sicher, dass sich während der Operation das kontaminierte Kühlmedium nicht in dem Bereich des knöchernen Nasengerüsts und des Gestells ansammelt und dass ein steriles Kühlmedium in den Bereich des knöchernen Nasengerüsts und des Gestells kontinuierlich gefördert werden kann. Das Nasengerüst kann dadurch effektiv gekühlt werden. Dadurch wird ein Absterben des Knochengewebes des Nasengerüsts durch eine Überhitzung verhindert. Ferner kann dadurch die Kühlvorrichtung im Betrieb das Operationswerkzeug effektiv kühlen. Dadurch kann der Verschleiß des Operationswerkzeugs weiter verringert werden.

### KURZE BESCHREIBUNG DER FIGUREN

Figur 1 ist eine schematische Querschnittsansicht einer Ausführungsform der Vorrichtung für eine Operation eines knöchernen Nasengerüsts der vorliegenden Erfindung.
Figur 2 ist eine schematische perspektivische Ansicht der Ausführungsform der Vorrichtung für eine Operation eines knöchernen Nasengerüsts der vorliegenden Erfindung gemäß Figur 1.
Figur 3 ist eine schematische Querschnittsansicht einer weiteren Ausführungsform der Vorrichtung für eine Operation eines knöchernen Nasengerüsts der vorliegenden Erfindung.
Figur 4 ist eine schematische perspektivische Ansicht der Ausführungsform der Vorrichtung für eine Operation eines knöchernen Nasengerüsts der vorliegenden Erfindung gemäß Figur 3.
Figur 5 ist eine schematische Querschnittsansicht einer weiteren Ausführungsform der Vorrichtung für eine Operation eines knöchernen Nasengerüsts der vorliegenden Erfindung.
Figur 6 ist eine schematische perspektivische Ansicht der Ausführungsform der Vorrichtung für eine Operation eines knöchernen Nasengerüsts der vorliegenden Erfindung gemäß Figur 5.
Figur 7 ist eine schematische Querschnittsansicht einer weiteren Ausführungsform der Vorrichtung für eine Operation eines knöchernen Nasengerüsts der vorliegenden Erfindung.
Figur 8 ist eine schematische perspektivische Ansicht der Ausführungsform der Vorrichtung für eine Operation eines knöchernen Nasengerüsts der vorliegenden Erfindung gemäß Figur 7.
Figur 9 ist eine schematische Querschnittsansicht einer weiteren Ausführungsform der Vorrichtung für eine Operation eines knöchernen Nasengerüsts der vorliegenden Erfindung.
Figur 10 ist eine schematische perspektivische Ansicht der Ausführungsform der Vorrichtung für eine Operation eines knöchernen Nasengerüsts der vorliegenden Erfindung gemäß Figur 9.
Figur 11 ist eine vergrößerte schematische Querschnittsansicht einer weiteren Ausführungsform der Vorrichtung für eine Operation eines knöchernen Nasengerüsts der vorliegenden Erfindung.
Figur 12 ist eine vergrößerte schematische Querschnittsansicht einer weiteren Ausführungsform der Vorrichtung für eine Operation eines knöchernen Nasengerüsts der vorliegenden Erfindung.

### DETAILLIERTE BESCHREIBUNG

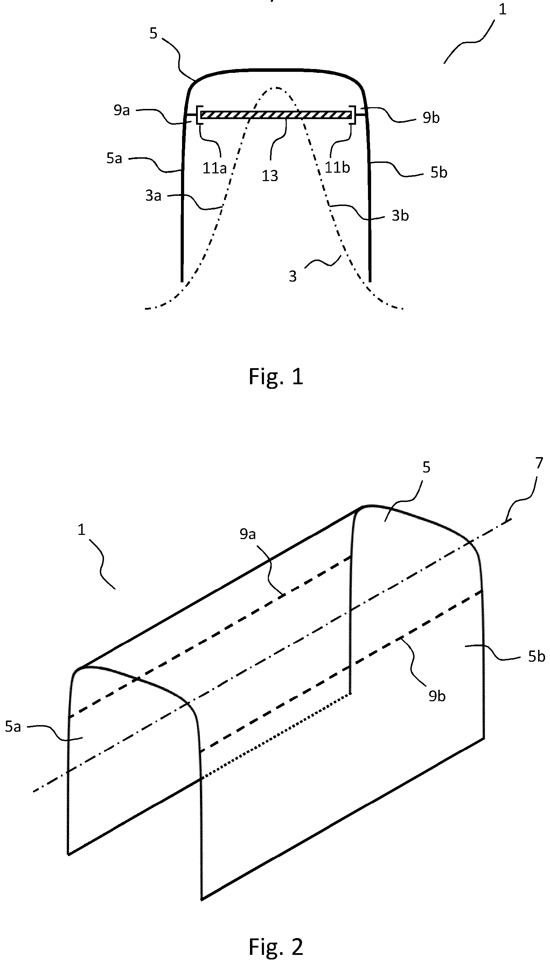

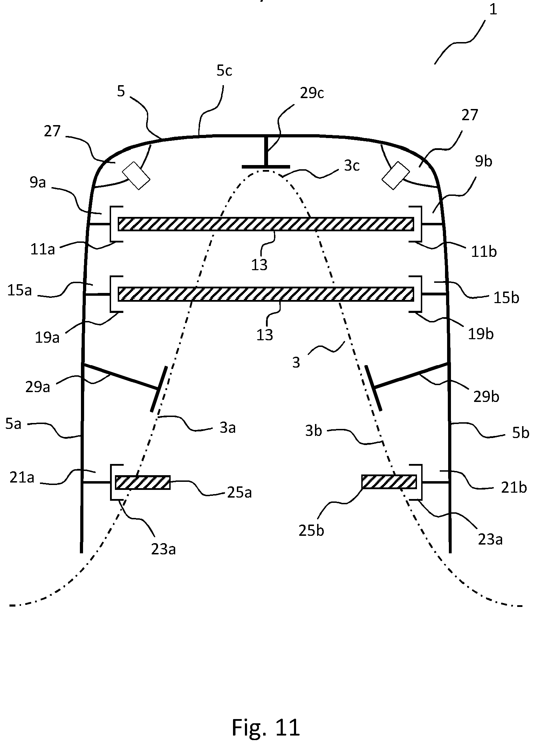

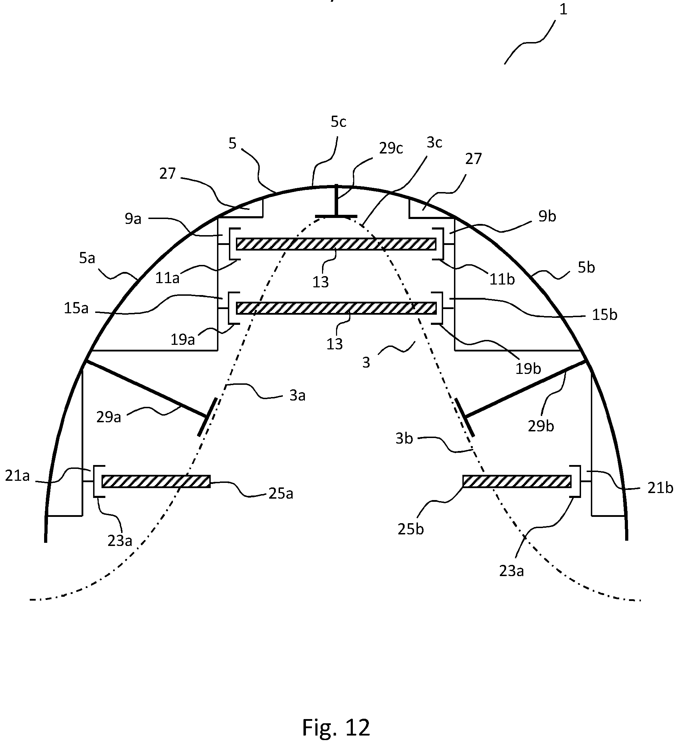

In den Figuren 1, 3, 5, 7 und 9 sind Operationswerkzeuge 13 oder 25a, 25b für ein besseres Verständnis der Erfindung und zur Veranschaulichung der Führung der Operationswerkzeuge 12, 25a, 25b in den Führungsmitteln dargestellt. Es ist zu beachten, dass in den Figuren 11 und 12 mehrere Operationswerkzeuge 13, 25a, 25b für ein besseres Verständnis der Erfindung und zur Veranschaulichung der Führung der Operationswerkzeuge 12, 25a, 25b in den Führungsmitteln dargestellt sind. Bevorzugt werden die Schnitte während der Operation jedoch nicht gleichzeitig, sondern in einer vorbestimmten Reihenfolge durchgeführt.

Die Figuren 1 und 2 zeigen eine Ausführungsform der Vorrichtung 1 für eine Operation eines knöchernen Nasengerüsts 3 gemäß der vorliegenden Erfindung, die ein Gestell 5 umfasst und eine im Wesentlichen umgekehrt u-förmige Querschnittsform aufweist, die sich während der Operation entlang einer Nasenrückenlängsachse 7 räumlich erstreckt und dabei zumindest einen Bereich des Nasengerüsts 3 umgibt. Das Gestell 5 weist eine erste Seite 5a und eine zweite Seite 5b auf, die einander gegenüberliegen. Ferner ist zumindest eine stirnseitig ausgerichtete Kante (nicht dargestellt) des Gestells 5 abgerundet. Das Gestell 5 weist ferner eine glatte und/oder beschichtete und/oder keimabweisende Oberfläche auf. Während der Operation ist die erste Seite 5a des Gestells 5 benachbart zu einem Nasenbein 3a des Nasengerüsts 3 und die zweite Seite 5b des Gestells 5 benachbart zu einem Nasenbein 3b des Nasengerüsts 3 angeordnet. Ferner ist an der ersten Seite 5a des Gestells 5 ein erstes Führungsmittel 9a und an der zweiten Seite 5b des Gestells 5 ein zweites Führungsmittel 9b angeordnet. Das erste Führungsmittel 9a und das zweite Führungsmittel 9b sind an einer inneren Oberfläche des Gestells 5 jeweils benachbart zu dem Nasenbein 3a und dem Nasenbein 3b des Nasengerüsts 3 angeordnet. Das erste Führungsmittel 9a und das zweite Führungsmittel 9b sind parallel zueinander und parallel zu der Nasenrückenlängsachse 7 angeordnet und bilden zusammen ein erstes Führungsmittelpaar 9a, 9b. Das erste Führungsmittel 9a und das zweite Führungsmittel 9b weisen jeweils zumindest eine erste Führungskontaktfläche 11a, 11b auf, die dazu eingerichtet ist, ein Operationswerkzeug 13 während der Operation zu einer zumindest bereichsweisen Durchtrennung des Nasengerüsts 3 in Richtung der Nasenrückenlängsachse 7 translatorisch zu führen.

Die im Wesentlichen umgekehrt u-förmige Form des Gestells 5 und die Dimension (Höhe, Breite, Länge) des Gestells 5 sind derart gestaltet, dass das Gestell 5 in eine für die Operation vorteilhafte Position zwischen der Nasenhaut (nicht dargestellt) und dem Nasengerüst 3 positioniert werden kann, ohne dass das Nasengerüst 3 oder die Nasenhaut dabei beschädigt werden. Zudem kann dadurch das Nasengerüst 3 und die Nasenhaut vor Verletzungen, z.B. durch das Operationswerkzeug 13, geschützt werden. Die Abrundung der stirnseitig ausgerichteten Kante schützt zudem die Nasenhaut und das Nasengerüst 3 bei der Einführung und der Entfernung der Vorrichtung 1. Durch die glatte und/oder beschichtete und/oder keimabweisende Oberfläche des Gestells 5 können im Betrieb Verletzungen der umgebenden Nasenhaut und des knöchernen Nasengerüsts 3 verhindert werden. Ferner wird dadurch verhindert, dass sich Keime an dem Gestell 5 ablagern, wodurch die Gefahr einer Infektion des Patienten verringert wird. Dadurch wird ermöglicht, dass die Vorrichtung nach dem Gebrauch gereinigt und für eine weitere Benutzung bei weiteren Operationen an anderen Patienten wiederverwendet werden kann. Das Auftreten von Komplikationen nach der Operation kann dadurch verringert werden. Die zumindest eine Führungskontaktfläche 11a, 11b der jeweiligen Führungsmittel 9a, 9b ermöglicht eine genaue translatorische Führung des Operationswerkzeugs 13 in Richtung der Nasenrückenlängsachse 7. Das erste Führungsmittelpaar 9a, 9b wird auf einer vorbestimmten Höhe bereitgestellt, so dass die geplanten Schnitte für die Veränderung der Form des Nasengerüsts 3 exakt durchgeführt werden können. Ferner ermöglicht die Bereitstellung des ersten Führungsmittelpaars 9a, 9b insbesondere auf einer exakten Höhe eine Beschleunigung und Vereinfachung des gesamten Operationsverlaufs, da auf eine manuelle Durchführung der Schnitte verzichtet werden kann und der Knochendeckel zur Abdeckung des "open roofs" am Ende der Operation nicht mehr eingearbeitet werden muss. Die geplanten Schnitte erfolgen nicht mehr "blind", sondern geführt entlang einer vorbestimmten Richtung des ersten Führungsmittelpaars 9a, 9b. Dadurch wird die Sicherheit der Durchführung der Operation erhöht. Ferner können dadurch operative Risiken, wie zum Beispiel ungewollte Knochenabsplitterungen, fehlerhafte Schnitte, etc. reduziert werden. Durch die benutzerfreundliche Ausgestaltung dieser Ausführungsform der Vorrichtung 1 für eine Operation eines knöchernen Nasengerüst 3 kann die benötigte Zeit für die Durchführung der Operation erheblich verringert werden. Dadurch kann die Gesundheitsbelastung für den Patienten reduziert werden, da die Zeit in einem narkotisierten Zustand verkürzt wird. Ferner können dadurch eine Kostenreduktion und eine Zeitersparnis sowohl für den Patienten als auch für den Operateur erreicht werden. Durch den einfachen Aufbau der Vorrichtung 1 für eine Operation eines knöchernen Nasengerüsts 3 ist die Vorrichtung 1 zudem robust und kostengünstig in der Herstellung.

Das in den Figuren 1 und 2 dargestellte erste Führungsmittelpaar 9a, 9b kann in einer Ausführungsform der vorliegenden Erfindung in einer Höhe relativ zu dem Gestell 5 verstellbar sein. Die Führungsmittel 9a und 9b sind dabei nicht fest mit dem Gestell verbunden, sondern als in einer Höhe verstellbare Führungsschienen 9a und 9b an dem Gestell 5 angebracht. Das erste Führungsschienenpaar 9a, 9b kann in einer vorbestimmten Höhe an dem Gestell 5 fixiert werden.

Durch die Verstellbarkeit des ersten Führungsschienenpaars 9a, 9b in einer Höhe können die aus der Gesichtsanalyse und Operationsplanung vorbestimmten Werte bzw. die vorbestimmte Form exakt auf das knöcherne Nasengerüst 3 übertragen werden und die Knochenschnitte können so durchgeführt werden, dass das Operationsergebnis exakt mit den geplanten Werten bzw. der geplanten Form übereinstimmen. Ferner können durch die Verstellbarkeit des ersten Führungsschienenpaars 9a, 9b in einer Höhe mehrere Schnitte in unterschiedlichen Höhen mit lediglich einem Führungsschienenpaar und einer Vorrichtung 1 durchgeführt werden. Dadurch wird die Flexibilität der Anwendung der Vorrichtung 1 erhöht. Ferner kann durch die benutzerfreundliche Ausgestaltung dieser Ausführungsform der Vorrichtung 1 für eine Operation eines knöchernen Nasengerüst 3 die benötigte Zeit für die Durchführung der Operation erheblich verringert werden. Dadurch kann die Gesundheitsbelastung für den Patienten reduziert werden, da die Zeit in einem narkotisierten Zustand verkürzt wird. Ferner kann dadurch eine Kostenreduktion sowohl für den Patienten als auch für den Operateur erreicht werden. Durch den einfachen Aufbau der Vorrichtung 1 für eine Operation eines knöchernen Nasengerüsts 3 ist die Vorrichtung 1 zudem robust und kostengünstig in der Herstellung.

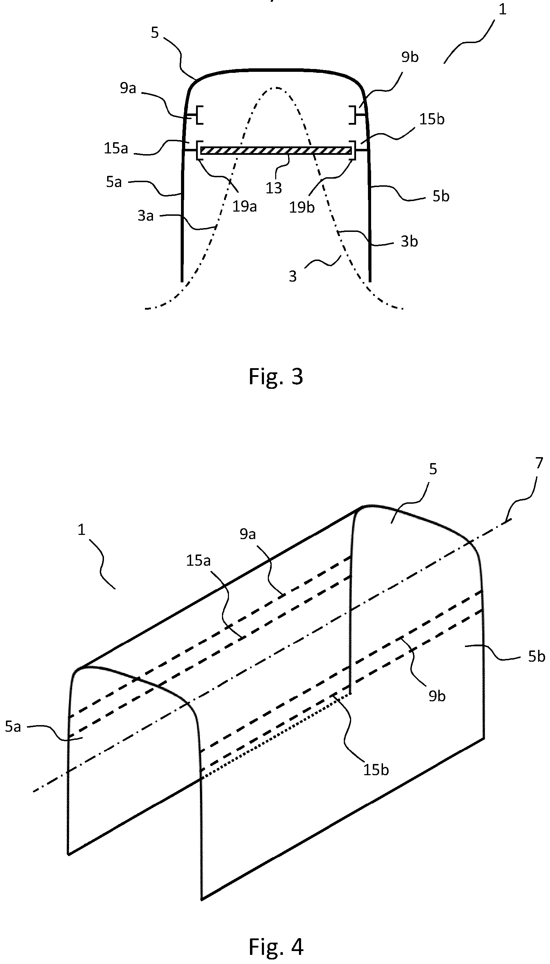

Die Figuren 3 und 4 zeigen eine weitere bevorzugte Ausführungsform der Vorrichtung 1 für eine Operation eines knöchernen Nasengerüsts 3 gemäß der vorliegenden Erfindung, die auf der in den Figuren 1 und 2 gezeigten Ausführungsform aufbaut. Auf eine wiederholende Beschreibung des Gestells 5, des ersten Führungsschienenpaars 9a, 9b und dessen Verstellbarkeit in einer Höhe wird daher im Folgenden verzichtet.

An der ersten Seite 5a des Gestells 5 ist ferner ein drittes Führungsmittel 15a und an der zweiten Seite 5b des Gestells 5 ein viertes Führungsmittel 15b angeordnet. Das dritte Führungsmittel 15a und das vierte Führungsmittel 15b sind in einer Höhe unterhalb des ersten Führungsmittels 9a und des zweiten Führungsmittels 9b angeordnet. Das dritte Führungsmittel 15a und das vierte Führungsmittel 15b sind an einer inneren Oberfläche des Gestells 5 jeweils benachbart zu dem Nasenbein 3a und dem Nasenbein 3b des Nasengerüsts 3 angeordnet. Das dritte Führungsmittel 15a und das vierte Führungsmittel 15b sind parallel zueinander und parallel zu der Nasenrückenlängsachse 7 angeordnet und bilden zusammen ein zweites Führungsmittelpaar 15a, 15b. Das dritte Führungsmittel 15a und das vierte Führungsmittel 15b weisen jeweils zumindest eine zweite Führungskontaktfläche 19a, 19b auf, die dazu eingerichtet ist, während der Operation ein Operationswerkzeug 13 zu einer zumindest bereichsweisen Durchtrennung des Nasengerüsts 3 in Richtung der Nasenrückenlängsachse 7 translatorisch zu führen.

Die zumindest eine Führungskontaktfläche 19a, 19b der jeweiligen Führungsmittel 15a, 15b ermöglicht eine genaue translatorische Führung des Operationswerkzeugs 13 in Richtung der Nasenrückenlängsachse 7. Das zweite Führungsmittelpaar 15a, 15b wird auf einer vorbestimmten Höhe bereitgestellt, so dass die geplanten Schnitte für die Veränderung der Form des Nasengerüsts 3 exakt durchgeführt werden können. Ferner ermöglicht die Bereitstellung des zweiten Führungsmittelpaars 15a, 15b insbesondere auf einer exakten Höhe eine Beschleunigung und Vereinfachung des gesamten Operationsverlaufs, da auf eine manuelle Durchführung der Schnitte verzichtet werden kann und der Knochendeckel zur Abdeckung des "open roofs" am Ende der Operation nicht mehr eingearbeitet werden muss. Die geplanten Schnitte erfolgen nicht mehr "blind", sondern geführt entlang einer vorbestimmten Richtung des zweiten Führungsmittelpaars 15a, 15b. Dadurch wird die Sicherheit der Durchführung der Operation erhöht. Ferner können dadurch operative Risiken wie zum Beispiel ungewollte Knochenabsplitterungen, fehlerhafte Schnitte, etc. reduziert werden. Durch die benutzerfreundliche Ausgestaltung dieser Ausführungsform der Vorrichtung 1 für eine Operation eines knöchernen Nasengerüst 3 kann die benötigte Zeit für die Durchführung der Operation erheblich verringert werden. Dadurch kann die Gesundheitsbelastung für den Patienten reduziert werden, da die Zeit in einem narkotisierten Zustand verkürzt wird. Ferner kann dadurch eine Kostenreduktion sowohl für den Patienten als auch für den Operateur erreicht werden. Durch den einfachen Aufbau der Vorrichtung 1 für eine Operation eines knöchernen Nasengerüsts 3 ist die Vorrichtung 1 zudem robust und kostengünstig in der Herstellung.

Das in den Figuren 3 und 4 dargestellte erste Führungsmittelpaar 9a, 9b und/oder das zweite Führungsmittelpaar 15a, 15b können in einer Ausführungsform der vorliegenden Erfindung in einer Höhe relativ zum Gestell 5 verstellbar sein. Die Führungsmittel 9a, 9b, 15a und 15b sind dabei nicht fest mit dem Gestell verbunden, sondern als in einer Höhe verstellbare Führungsschienen 9a, 9b, 15a, 15b an dem Gestell 5 angebracht. Das erste Führungsschienenpaar 9a, 9b und/oder zweite Führungsschienenpaar 15a, 15b können in einer vorbestimmten Höhe an dem Gestell 5 fixiert werden.

Durch die Verstellbarkeit des ersten Führungsschienenpaars 9a, 9b und/oder des zweiten Führungsschienenpaars 15a, 15b in einer Höhe können die aus der Gesichtsanalyse und Operationsplanung vorbestimmten Werte bzw. die vorbestimmte Form exakt auf das knöcherne Nasengerüst 3 übertragen werden und die Knochenschnitte können so durchgeführt werden, dass das Operationsergebnis exakt mit den geplanten Werten bzw. der geplanten Form übereinstimmen. Ferner können durch die Verstellbarkeit des ersten Führungsschienenpaars 9a, 9b und/oder des zweiten Führungsschienenpaars 15a, 15b in einer Höhe mehrere Schnitte in unterschiedlichen Höhen durchgeführt werden, ohne dass die Vorrichtung 1 entfernt und die Höhe der Führungsschienenpaare 9a, 9b und 15a, 15b neu eingestellt werden muss. Dadurch wird die Flexibilität der Anwendung der Vorrichtung erhöht. Ferner kann durch die benutzerfreundliche Ausgestaltung dieser Ausführungsform der Vorrichtung 1 für eine Operation eines knöchernen Nasengerüst 3 die benötigte Zeit für die Durchführung der Operation erheblich verringert werden. Dadurch kann die Gesundheitsbelastung für den Patienten reduziert werden, da die Zeit in einem narkotisierten Zustand verkürzt wird. Ferner kann dadurch eine Kostenreduktion sowohl für den Patienten als auch für den Operateur erreicht werden. Durch den einfachen Aufbau der Vorrichtung 1 für eine Operation eines knöchernen Nasengerüsts 3 ist die Vorrichtung 1 zudem robust und kostengünstig in der Herstellung.

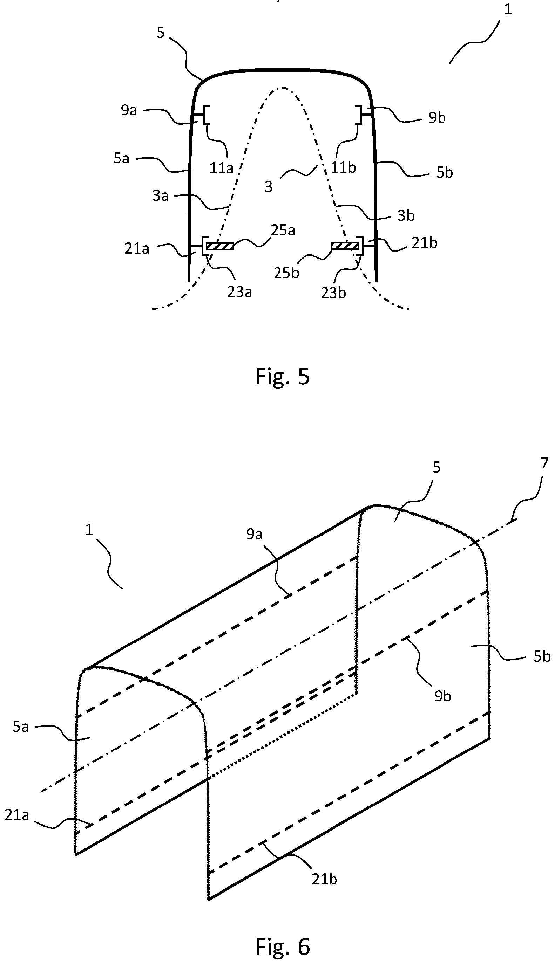

Die Figuren 5 und 6 zeigen eine weitere bevorzugte Ausführungsform der Vorrichtung 1 für eine Operation eines knöchernen Nasengerüsts 3 gemäß der vorliegenden Erfindung, die auf der in den Figuren 1 und 2 gezeigten Ausführungsform aufbaut. Es ist zu beachten, dass die anhand der Figuren 5 und 6 beschriebene Ausführungsform gleichermaßen auf der in den Figuren 3 und 4 gezeigten Ausführungsform oder auf einer Kombination der in den Figuren 1 bis 4 gezeigten Ausführungsformen aufbauen kann. Auf eine wiederholende Beschreibung des Gestells 5, des ersten Führungsmittelpaars 9, des zweiten Führungsmittelpaars 15a, 15b und deren Verstellbarkeit in einer Höhe wird daher im Folgenden verzichtet.

An der ersten Seite 5a des Gestells 5 ist ferner ein fünftes Führungsmittel 21a und an der zweiten Seite 5b des Gestells 5 ein sechstes Führungsmittel 21b angeordnet. Das fünfte Führungsmittel 21a und das sechste Führungsmittel 21b sind in einer Höhe unterhalb des ersten Führungsmittels 9a und des zweiten Führungsmittel 9b sowie unterhalb des dritten Führungsmittels 15a (nicht dargestellt in den Figuren 5 und 6) und des vierten Führungsmittels 15b (nicht dargestellt in den Figuren 5 und 6) angeordnet. Das fünfte Führungsmittel 21a und das sechste Führungsmittel 21b sind an einer inneren Oberfläche des Gestells 5 jeweils benachbart zu dem Nasenbein 3a und dem Nasenbein 3b des Nasengerüsts 3 angeordnet. Das fünfte Führungsmittel 21a und das sechste Führungsmittel 21b sind parallel zueinander und parallel zu der Nasenrückenlängsachse 7 angeordnet. Im Gegensatz zu den darüber angeordneten Führungsmitteln 9a, 9b, 15a, 15b bilden das fünfte Führungsmittel 21a und das sechste Führungsmittel 21b kein Führungsmittelpaar. Das fünfte Führungsmittel 21a und das sechste Führungsmittel 21b weisen jeweils zumindest eine dritte Führungskontaktfläche 23a, 23b auf, die dazu eingerichtet ist, während der Operation jeweils ein Operationswerkzeug 25a, 25b zu einer zumindest bereichsweisen Durchtrennung eines Nasenbeins 3a oder eines Nasenbeins 3b des Nasengerüsts 3 in Richtung der Nasenrückenlängsachse 7 translatorisch zu führen. Bei den Operationswerkzeugen 25a, 25b handelt es sich um eine andere Art von Operationswerkzeug als das Operationswerkzeug 13. Wie in Figur 5 dargestellt ist, erstrecken sich die Operationswerkzeuge 25a, 25b nicht über die gesamte Breite zwischen dem fünften Führungsmittel 21a und dem sechsten Führungsmittel 21b. Dadurch wird sichergestellt, dass während der Operation lediglich das Nasenbein 3a von dem Operationswerkzeug 25a zumindest bereichsweise durchtrennt wird und lediglich das Nasenbein 3b von dem Operationswerkzeug 25b zumindest bereichsweise durchtrennt wird.

Die zumindest eine Führungskontaktfläche 23a des fünften Führungsmittels 21a und die zumindest eine Führungskontaktfläche 23b des sechsten Führungsmittels 21b ermöglichen eine genaue translatorische Führung der Operationswerkzeuge 25a, 25b in Richtung der Nasenrückenlängsachse 7. Das fünfte Führungsmittel 21a und das sechste Führungsmittel 21b werden in einer Ausführungsform der vorliegenden Erfindung auf einer vorbestimmten Höhe bereitgestellt, so dass die geplanten Schnitte für die Veränderung der Form des Nasengerüsts 3 exakt durchgeführt werden können. Ferner ermöglicht die Bereitstellung des fünften Führungsmittels 21a und des sechsten Führungsmittels 21b insbesondere auf einer exakten Höhe eine Beschleunigung und Vereinfachung des gesamten Operationsverlaufs, da auf eine manuelle Durchführung der Schnitte verzichtet werden kann und der Knochendeckel zur Abdeckung des "open roofs" am Ende der Operation nicht mehr eingearbeitet werden muss. Die geplanten Schnitte erfolgen nicht mehr "blind", sondern geführt entlang einer vorbestimmten Richtung des fünften Führungsmittels 21a und des sechsten Führungsmittels 21b. Dadurch wird die Sicherheit der Durchführung der Operation erhöht. Ferner können dadurch operative Risiken wie zum Beispiel ungewollte Knochenabsplitterungen, fehlerhafte Schnitte, etc. reduziert werden. Durch die benutzerfreundliche Ausgestaltung dieser erfindungsgemäßen Ausführungsform der Vorrichtung 1 für eine Operation eines knöchernen Nasengerüst 3 kann die benötigte Zeit für die Durchführung der Operation erheblich verringert werden. Dadurch kann die Gesundheitsbelastung für den Patienten reduziert werden, da die Zeit in einem narkotisierten Zustand verkürzt wird. Ferner kann dadurch eine Kostenreduktion sowohl für den Patienten als auch für den Operateur erreicht werden. Durch den einfachen Aufbau der Vorrichtung 1 für eine Operation eines knöchernen Nasengerüsts 3 ist die Vorrichtung 1 zudem robust und kostengünstig in der Herstellung.

Das in den Figuren 5 und 6 dargestellte erste Führungsmittelpaar 9a, 9b und/oder das fünfte Führungsmittel 21a und das sechste Führungsmittel 21b können in einer Ausführungsform in einer Höhe relativ zum Gestell 5 verstellbar sein. Zudem kann bei einer Ausführungsform der vorliegenden Erfindung, die zudem das zweite Führungsmittelpaar 15a, 15b beinhaltet, auch das zweite Führungsmittelpaar 15a, 15b in einer Höhe relativ zum Gestell 5 verstellbar sein. Die in den Figuren 5 und 6 dargestellten Führungsmittel 9a, 9b, 21a und 21b sind dabei nicht fest mit dem Gestell verbunden, sondern als in einer Höhe verstellbare Führungsschienen 9a, 9b, 21a, 21b an dem Gestell 5 angebracht. Das erste Führungsschienenpaar 9a, 9b und/oder die fünfte Führungsschiene 21a und die sechste Führungsschiene 21b können in einer vorbestimmten Höhe an dem Gestell 5 fixiert werden.

Durch die Verstellbarkeit der fünften Führungsschiene 21a und der sechsten Führungsschiene 21b in einer Höhe können die aus der Gesichtsanalyse und Operationsplanung vorbestimmten Werte bzw. die vorbestimmte Form exakt auf das knöcherne Nasengerüst 3 übertragen werden und die Knochenschnitte können so durchgeführt werden, dass das Operationsergebnis exakt mit den geplanten Werten bzw. der geplanten Form übereinstimmen. Ferner können durch die Verstellbarkeit der fünften Führungsschiene 21a und der sechsten Führungsschiene 21b in einer Höhe mehrere Schnitte in unterschiedlichen Höhen mit lediglich zwei Führungsschienen und einer Vorrichtung durchgeführt werden. Dadurch wird die Flexibilität der Anwendung der Vorrichtung erhöht. Ferner kann durch die benutzerfreundliche Ausgestaltung dieser Ausführungsform der Vorrichtung 1 für eine Operation eines knöchernen Nasengerüst 3 die benötigte Zeit für die Durchführung der Operation erheblich verringert werden. Dadurch kann die Gesundheitsbelastung für den Patienten reduziert werden, da die Zeit in einem narkotisierten Zustand verkürzt wird. Ferner kann dadurch eine Kostenreduktion sowohl für den Patienten als auch für den Operateur erreicht werden. Durch den einfachen Aufbau der Vorrichtung 1 für eine Operation eines knöchernen Nasengerüsts 3 ist die Vorrichtung 1 zudem robust und kostengünstig in der Herstellung.

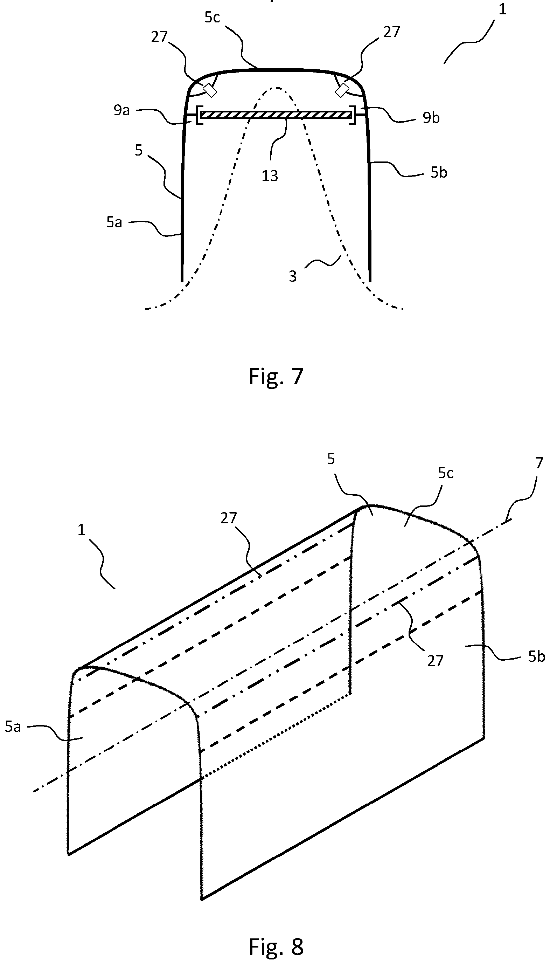

Die Figuren 7 und 8 zeigen eine weitere bevorzugte Ausführungsform der Vorrichtung 1 für eine Operation eines knöchernen Nasengerüsts 3 gemäß der vorliegenden Erfindung, die auf der in den Figuren 1 und 2 gezeigten Ausführungsform aufbaut. Es ist zu beachten, dass die anhand der Figuren 7 und 8 beschriebene Ausführungsform gleichermaßen auf der in den Figuren 3 und 4 und/oder der in den Figuren 5 und 6 gezeigten Ausführungsform der vorliegenden Erfindung aufbauen kann. Auf eine wiederholende Beschreibung des Gestells 5, des ersten Führungsmittelpaars 9a, 9b, des zweiten Führungsmittelpaars 15a, 15b, des fünften Führungsmittels 21a und des sechsten Führungsmittels 21b und deren Verstellbarkeit in einer Höhe wird daher im Folgenden verzichtet.

In der in den Figuren 7 und 8 gezeigten Ausführungsform ist in dem Gestell 5 oder benachbart zu dem Gestell 5 der Vorrichtung 1 für eine Operation eines knöchernen Nasengerüsts 3 ferner eine Kühlvorrichtung 27 bereitgestellt. In der in den Figuren 7 und 8 dargestellten erfindungsgemäßen Ausführungsform ist die Kühlvorrichtung 27 als Kanalstruktur in einem Übergangsbereich zwischen einer jeweiligen Seite 5a, 5b des Gestells 5 und einem dachförmigen Abschnitt 5c des Gestells 5 ausgeführt. Es ist zu beachten, dass die vorliegende Erfindung nicht auf diese Anordnung der Kühlvorrichtung 27 beschränkt ist. Auch Ausführungsformen mit nur einer oder mehreren Kühlvorrichtungen 27 sind möglich. Ferner ist die vorliegende Erfindung nicht darauf beschränkt, dass die Kühlvorrichtung 27 in dem in den Figuren 7 und 8 dargestellten Bereich angeordnet ist. Ebenfalls sind Anordnungen in anderen Bereichen an oder in dem Gestell 5, die eine effiziente und automatisierte Kühlung ermöglichen, denkbar. Die Kühlvorrichtung 27 ist dazu eingerichtet, zumindest einen zu der oder den Führungsmitteln benachbarten Bereich der Vorrichtung 1 zu kühlen. Insbesondere ist die Kühlvorrichtung 27 dazu eingerichtet, während der Operation den Kontaktbereich zwischen dem Operationswerkzeug 13 und dem Nasengerüst 3 zu kühlen und dabei entstehende Knochensplitter aus dem Kontaktbereich abzutragen. Die Kühlvorrichtung 27 ist in einer weiteren erfindungsgemäßen Ausführungsform ebenfalls dazu eingerichtet, die Operationswerkzeuge 25a, 25b und den Kontaktbereich zwischen den Operationswerkzeugen 25a, 25b und dem Nasengerüst 3 gleichermaßen vorteilhaft zu kühlen.

Durch die Kühlvorrichtung 27 kann während der Operation das Nasengerüst 3 und insbesondere der Kontaktbereich zwischen dem Operationswerkzeug 13 und dem Nasengerüst 3 effektiv und automatisiert gekühlt werden. Dadurch wird ein Absterben des Knochengewebes des Nasengerüsts 3 verhindert. Ein entnommener und bearbeiteter Teil des Nasengerüsts 3 bleibt somit intakt und kann replantiert werden. Die Wahrscheinlichkeit, dass der zu replantierende Teil des Nasengerüsts 3 vom Körper abgestoßen wird, wird dadurch verringert. Ferner kann durch die Kühlvorrichtung 27 während der Operation das Operationswerkzeug 13 effektiv und automatisiert gekühlt werden. Dadurch kann der Verschleiß des Operationswerkzeugs 13 verringert werden.

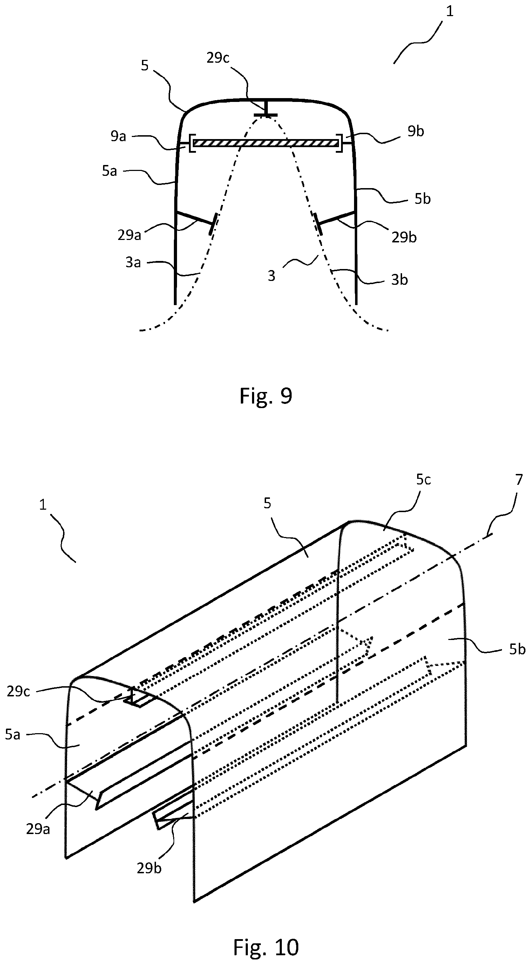

Die Figuren 9 und 10 zeigen eine weitere bevorzugte Ausführungsform der Vorrichtung 1 für eine Operation eines knöchernen Nasengerüsts 3 gemäß der vorliegenden Erfindung, die auf der in den Figuren 1 und 2 gezeigten Ausführungsform aufbaut. Es ist zu beachten, dass die anhand der Figuren 9 und 10 beschriebene Ausführungsform gleichermaßen auf der in den Figuren 3 und 4 und/oder der in den Figuren 5 und 6 und/oder der in den Figuren 7 und 8 gezeigten Ausführungsform der vorliegenden Erfindung aufbauen kann. Auf eine wiederholende Beschreibung des Gestells 5, des ersten Führungsmittelpaars 9, des zweiten Führungsmittelpaars 15a, 15b, des fünften Führungsmittels 21a und des sechsten Führungsmittels 21b, deren Verstellbarkeit in einer Höhe und der Kühlvorrichtung 27 wird daher im Folgenden verzichtet.

In der in den Figuren 9 und 10 gezeigten Ausführungsform ist in oder an dem Gestell 5 der Vorrichtung 1 für eine Operation eines knöchernen Nasengerüsts 3 ferner zumindest ein Fixierungsmittel bereitgestellt, das an der ersten Seite 5a oder der zweiten Seite 5b oder einem die erste und die zweite Seite verbindenden dachförmigen Abschnitt 5c des Gestells 5 nach innen ausgerichtet angeordnet und dazu eingerichtet ist, das Gestell 5 während der Operation relativ zu dem knöchernen Nasengerüst 3 zu positionieren und daran zu fixieren. In einer bevorzugten erfindungsgemäßen Ausführungsform umfasst die Vorrichtung 1 für eine Operation eines knöchernen Nasengerüsts 3 drei Fixierungsmittel 29a, 29b, 29c. Das Fixierungsmittel 29a ist an der ersten Seite 5a des Gestells 5 angeordnet. Das Fixierungsmittel 29b ist an der zweiten Seite 5b des Gestells 5 angeordnet. Das Fixierungsmittel 29c ist an dem dachförmigen Abschnitt 5c des Gestells 5 angeordnet. In einer weiteren bevorzugten erfindungsgemäßen Ausführungsform sind die Fixierungsmittel 29a, 29b, 29c als Schrauben oder Stifte ausgeführt.

Durch die Bereitstellung des oder der Fixierungsmittel 29a, 29b, 29c kann das Gestell 5 in einer exakten Position relativ zu dem Nasengerüst 3 angeordnet und fixiert werden. Dies ermöglicht die Durchführung von exakten Schnitten an dem Nasengerüst 3 während der Operation. Ferner kann dadurch verhindert werden, dass sich die Vorrichtung 1 während der Durchführung der Schnitte an dem Nasengerüst 3 ungewollt verschiebt. Durch die Bereitstellung von zumindest drei Fixierungsmitteln 29a, 29b, 29c kann das Gestell 5 in einer exakten Position relativ zu dem Nasengerüst 3 angeordnet und fixiert werden und die Belastung auf das Nasengerüst 3 durch Kräfte, die über die Fixierungsmittel 29a, 29b, 29c auf das Nasengerüst 3 übertragen werden, kann reduziert werden. Dies ermöglicht die Durchführung von exakten Schnitten an dem Nasengerüst 3 und vermindert das Risiko einer Verletzung des Nasengerüsts 3 durch die Vorrichtung 1. Ferner kann dadurch verhindert werden, dass sich die Vorrichtung 1 während der Durchführung der Schnitte an dem Nasengerüst 3 ungewollt verschiebt. Zudem kann durch die Bereitstellung von Schrauben oder Stiften als Fixierungsmittel 29a, 29b, 29c das Gestell 5 einfach in einer exakten Position relativ zu dem Nasengerüst 3 angeordnet und fixiert werden.

Die Figuren 11 und 12 zeigen Ausführungsformen des Systems 10 zur Operation eines knöchernen Nasengerüsts 3 gemäß der vorliegenden Erfindung, die auf einer Kombination der in den Figuren 1 bis 10 gezeigten Ausführungsformen mit den Operationswerkzeugen 13, 25a, 25b und einer Absaugvorrichtung (nicht gezeigt) aufbaut. Die in Figur 12 gezeigte Ausführungsform der Vorrichtung 1 vorliegenden Erfindung unterscheidet sich von den in Figuren 1 bis 11 gezeigten Ausführungsform lediglich in der Form des Gestells. Auf eine wiederholende Beschreibung des Gestells 5, des ersten Führungsmittelpaars 9a, 9b, des zweiten Führungsmittelpaars 15a, 15b, des fünften Führungsmittels 21a und des sechsten Führungsmittels 21b, deren Verstellbarkeit in einer Höhe, der Kühlvorrichtung 27 und der Fixierungsmittel 29a, 29b, 29c wird daher im Folgenden verzichtet.

Die Operationswerkzeuge 13, 25a, 25b in einer Ausführungsform der vorliegenden Erfindung können beispielsweise als ein Meißel, eine oszillierende Säge oder ein Piezohandstück ausgeführt sein.

Diese vorteilhaften Ausführungsformen der Operationswerkzeuge 13, 25a, 25b können die für die Operation erforderlichen Schnitte vorteilhaft mit der Vorrichtung für die Operation eines knöchernen Nasengerüsts durchgeführt werden.

Ferner kann die Kühlvorrichtung 27 in einer Ausführungsform der vorliegenden Erfindung in den Operationswerkzeugen 13, 25a, 25b integriert sein. Die Operationswerkzeuge 13, 25a, 25b werden dabei mit einem Kühlmedium (nicht dargestellt) durchströmt und können das Kühlmedium in den Schneidbereich und in einen Kontaktbereich zwischen den Operationswerkzeugen 13, 25a, 25b und dem Nasengerüst 3 einbringen.

Durch die in den Operationswerkzeugen 13, 25a, 25b integrierte Kühlvorrichtung 27 kann das Nasengerüst 3 und insbesondere der Kontaktbereich zwischen dem oder den Operationswerkzeugen 13, 25a, 25b während der Operation effektiv und automatisiert gekühlt werden. Dadurch wird ein Absterben des Knochengewebes des Nasengerüsts 3 verhindert. Ein entnommener und bearbeiteter Teil des Nasengerüsts 3 bleibt somit intakt und kann replantiert werden. Die Wahrscheinlichkeit, dass der zu replantierende Teil des Nasengerüsts 3 vom Körper abgestoßen wird, wird dadurch verringert. Ferner kann durch die in den Operationswerkzeugen 13, 25a, 25b integrierte Kühlvorrichtung 27 während der Operation das oder die Operationswerkzeuge 13, 25a, 25b effektiv und automatisiert gekühlt werden. Dadurch kann der Verschleiß des oder der Operationswerkzeuge 13, 25a, 25b verringert werden.

In einer weiteren Ausführungsform der vorliegenden Erfindung umfasst das System für eine Operation eines knöchernen Nasengerüsts ferner eine Absaugvorrichtung (nicht dargestellt), die an der Vorrichtung 1 oder an dem oder den Operationswerkzeugen 13, 25a, 25b angeordnet ist und dazu eingerichtet ist, das Kühlmedium während der Operation aus einer Umgebung des Gestells 5 und des knöchernen Nasengerüsts 3 abzusaugen.

Durch die Bereitstellung der Absaugvorrichtung kann das während der Operation verwendete Kühlmedium, das beispielsweise durch Blut oder Knochensplitter kontaminiert wurde, aus einem Bereich des Nasengerüsts 3 entfernt werden. Dadurch kann sichergestellt werden, dass sich das kontaminierte Kühlmedium nicht in dem Bereich des knöchernen Nasengerüsts 3 ansammelt und dass ein steriles Kühlmedium in den Bereich des Nasengerüsts 3 gefördert werden kann. Das Nasengerüst 3 kann dadurch effektiver gekühlt werden. Dadurch kann ein Absterben des Knochengewebes des Nasengerüsts 3 besser verhindert werden. Ferner kann dadurch die Kühlvorrichtung 27 im Betrieb das oder die Operationswerkzeuge 13, 25a, 25b effektiver kühlen. Dadurch kann der Verschleiß des oder der Operationswerkzeuge 13, 25a, 25b weiter verringert werden.

Nachfolgend wird anhand der Figuren 11 und 12 die Funktionalität der einzelnen Elemente der Vorrichtung 1 und des Systems 10 anhand eines Anwendungsbeispiels detailliert beschrieben. Die in diesem Anwendungsbeispiel beschriebene Ausführungsform umfasst als Führungsmitteln in einer Höhe verstellbare Führungsschienen 9a, 9b, 15a, 15b, 21a, 21b.

Die vorstehend beschriebene Vorrichtung 1 bildet zusammen mit den Operationswerkzeugen 13, 25a, 25b das System 10 für eine Operation eines knöchernen Nasengerüsts 3. In Abhängigkeit der Ausführungsform der vorliegenden Erfindung kann das System 10 mit dem Operationswerkzeugen 13 und/oder den Operationswerkzeugen 25a, 25b bereitgestellt sein. Die Operationswerkzeuge 13, 25a, 25b sind dazu eingerichtet, das Nasengerüst 3 zumindest bereichsweise im Wesentlichen längs der Nasenrückenlängsachse 7 zu durchtrennen. Die Operationswerkzeuge 13, 25a, 25b können beispielsweise als ein Meißel, eine oszillierende Säge oder ein Piezohandstück ausgeführt sein. Damit können die für die Operation eines Nasengerüsts 3 erforderlichen Schnitte vorteilhaft mit der Vorrichtung 1 durchgeführt werden. Vor der Operation wird bei der Gesichtsanalyse und Operationsplanung eine bestimmte Formänderung des Nasengerüsts 3 (z.B. die Abtragung eines Nasenhöckers oder die Absenkung des Nasenrückens 3c um z.B. 3 mm) als die ästhetisch vorteilhafteste Veränderung ermittelt, mit dem Patienten besprochen und geplant. Häufig werden dabei Computersimulationen angewendet. Die Patienten werden fotografiert und das Foto wird auf einem Computer mit entsprechenden Simulationsprogrammen bearbeitet, um dem Patienten eine Vorstellung davon zu vermitteln, wie die Nase nach der Operation aussehen wird. Die aus der Computersimulation erhaltenen Daten können bevorzugt Höhenangaben enthalten, auf die die Führungsschienen 9a, 9b, 15a, 15b, 21a, 21b eingestellt und an dem Gestell 5 fixiert werden. Ferner enthalten die aus der Computersimulation erhaltenen Daten Informationen, wie ein Nasenrücken 3c des Nasengerüsts 3 bearbeitet werden muss, um die ästhetisch vorteilhafteste Veränderung zu erreichen. Damit ist die Operationsvorbereitung im Wesentlichen abgeschlossen. Zu Beginn der Operation wird zunächst die Nasenhaut von dem knöchernen Nasengerüst 3 abgelöst und die Vorrichtung 1 zwischen der Nasenhaut und dem Nasengerüst 3 eingeschoben. Mit Hilfe von festgelegten Fixierungspunkten an dem Nasengerüst 3 wird die Vorrichtung 1 exakt relativ zu dem Nasengerüst 3 positioniert und über die Fixierungsmittel 29a, 29b, 29c daran fixiert. Nachfolgend wird mit einem ersten Schnitt mit dem Operationswerkzeug 13, das in dem ersten Führungsschienenpaar 9a, 9b translatorisch in Richtung der Nasenrückenlängsachse 7 geführt wird, der Nasenrücken 3c des Nasengerüsts 3 mit einer definierten Knochendicke abgetragen. Der durchgetrennte Teil des Nasengerüsts 3 wird anschließend entfernt. In anderen Worten wird, in Abhängigkeit der gewählten Operationstechnik, ein deckelförmiger Teil des Nasengerüsts 3 durchgetrennt und entfernt, wodurch ein nach oben hin offener Bereich (ein so genanntes "open roof") an dem Nasengerüst 3 entsteht. Der deckelförmige Teil des Nasengerüsts 3 kann am Ende der Operation, je nach gewählter Operationstechnik, zum Verschluss des "open roofs" wieder zurückgelegt, also replantiert werden. Mit einem zweiten Schnitt mit dem Operationswerkzeug 13, das in dem zweiten Führungsschienenpaar 15a, 15b geführt wird, wird ein streifenförmiger Teil in einer definierten Höhe an dem Nasenbein 3a und dem Nasenbein 3b des Nasengerüsts 3 abgetrennt und entfernt. Dadurch kann das Nasengerüst 3 um eine aus der Computersimulation ermittelte Höhe erniedrigt werden. Wahlweise werden mit zwei weiteren Schnitten mit den Operationswerkzeugen 25a, 25b jeweils das Nasenbein 3a und das Nasenbein 3b des Nasengerüsts 3 an ihrer Basis durchtrennt. Diese Schnitte ermöglichen eine Mobilisierung des Nasenbeins 3a und des Nasenbeins 3b des Nasengerüsts 3. Diese können dadurch in einer Position und in einem Abstand zueinander eingestellt werden. Während jedem der durchgeführten Schnitte kann ein Kühlmedium (nicht dargestellt) über die Kühlvorrichtung 27 zu den Operationswerkzeugen 13, 25a, 25b und einem zu durchtrennenden benachbarten Bereich an dem Nasengerüst 3 gefördert werden. Das Kühlmedium kann dadurch während der Durchführung der Schnitte während der Operation die Operationswerkzeuge 13, 25a, 25b sowie die zu schneidenden benachbarten Bereiche an dem Nasengerüst 3 effektiv kühlen. Zudem können über das Kühlmedium die während den Schnitten entstehenden Knochensplitter abgetragen werden. Das Kühlmedium kann anschließend über eine Absaugvorrichtung (nicht dargestellt) aus dem Bereich des Nasengerüsts 3 und des Gestells 5 abgesaugt werden. Anschließend werden die Fixierungsmittel 29 von dem Nasengerüst 3 gelöst und die Vorrichtung 1 wird entfernt. Danach können das Nasenbein 3a und das Nasenbein 3b des Nasengerüsts 3 in einer Position, beispielsweise bei einem Schiefstand des Nasengerüsts zu einer Begradigung der Nasenrückenlängsachse, und in ihrem Abstand zueinander, beispielsweise zu einer Verschmälerung der Nase bei einer verbreiterten Nasenbasis, eingestellt werden und der in einer definierten Dicke abgetragene Nasenrücken 3c kann zum Verschluss des "open roofs" replantiert werden.

Wird vom Operateur in geeigneten Fällen eine sogenannte "push down" Operationstechnik gewählt, bei dem der Nasenrücken 3c erhalten und in situ belassen wird, können durch die Vorrichtung 1 seitlich an den Nasenbeinen 3a, 3b jeweils zwei Knochenschnitte in einer exakten Position und mit einem exakten Abstand zueinander, der dem Betrag der geplanten Erniedrigung des Nasenrückens 3c und damit der Absenkung des Nasenprofils entspricht, durchgeführt werden. Die so umschnittenen Knochenstreifen werden dann auf beiden Seiten des Nasengerüsts entfernt und der in situ belassene Nasenrücken 3c hinuntergedrückt (sogenanntes "push down"). Der Nasenrücken kommt damit genau auf dem um die Knochenstreifen beidseitig erniedrigten Nasengerüst zu liegen, wodurch ein exaktes Operationsergebnis erzielt wird.

Weitere vorteilhafte Ausgestaltungen und Abwandlungen ergeben sich für den Fachmann aus den hier beschriebenen Ausführungsbeispielen und werden von ihm als zur Erfindung gehörig verstanden.

Die vorliegende Erfindung wurde basierend auf den vorstehenden Ausführungsformen beschrieben, aber die vorliegende Erfindung ist nicht auf die vorstehend beschriebenen Ausführungsformen beschränkt, und verschiedene Modifikationen und Änderungen sind basierend auf der technischen Idee der vorliegenden Erfindung möglich.

In einer weiteren bevorzugten Ausführungsform der vorliegenden Erfindung sind die Führungsmittel jeweils als Aussparung an einer Innenseite der ersten oder der zweiten Seite des Gestells ausgeführt. Vorzugsweise sind die Aussparungen als gefräste oder eingeschliffene Rillen an der jeweiligen Innenseite der ersten oder der zweiten Seite des Gestells ausgeführt. In einer weiteren Ausführungsform sind mehrere dieser Rillen in unterschiedlichen Höhen in dem Gestell bereitgestellt. Vorteilhaft ist auch die Bereitstellung einer Vielzahl dieser Vorrichtungen mit Rillen in unterschiedlichen, exakt definierten Höhen.

In einer weiteren bevorzugten Ausführungsform werden die Operationswerkzeuge als re-sterilisierbare und damit wieder verwendbare Operationswerkzeuge oder als einmal verwendbare Operationswerkzeuge bereitgestellt.

### BEZUGSZEICHENLISTE

- 1: Vorrichtung für eine Operation eines knöchernen Nasengerüsts
- 3: Nasengerüst
- 3a: Nasenbein
- 3b: Nasenbein
- 3c: Nasenrücken
- 5: Gestell
- 5a: Erste Seite
- 5b: Zweite Seite
- 5c: Dachförmiger Abschnitt
- 7: Nasenrückenlängsachse
- 9a: Erstes Führungsmittel
- 9b: Zweites Führungsmittel
- 10: System für eine Operation eines knöchernen Nasengerüsts
- 11a, 11b: Erste Führungskontaktfläche
- 13: Operationswerkzeug
- 15a: Drittes Führungsmittel
- 15b: Viertes Führungsmittel
- 19a, 19b: Zweite Führungskontaktfläche
- 21a: Fünftes Führungsmittel
- 21b: Sechstes Führungsmittel
- 23a, 23b: Dritte Führungskontaktfläche
- 25a: Operationswerkzeug
- 25b: Operationswerkzeug
- 27: Kühlvorrichtung
- 29a: Erstes Fixierungsmittel
- 29b: Zweites Fixierungsmittel
- 29c: Drittes Fixierungsmittel

## Patentansprüche

1. Vorrichtung (1) für eine Operation eines knöchernen Nasengerüsts (3), umfassend:
ein Gestell (5), das eine im Wesentlichen umgekehrt u-förmige Form aufweist, während der Operation entlang einer Nasenrückenlängsachse (7) ausgerichtet ist und dabei zumindest einen Bereich des knöchernen Nasengerüsts (3) umgibt;
wobei das Gestell (5) eine erste und eine zweite Seite (5a, 5b) aufweist, die einander gegenüberliegend und während der Operation jeweils benachbart zu einer entsprechenden Seite des knöchernen Nasengerüsts (3) angeordnet sind;
ein an der ersten Seite (5a) des Gestells (5) angeordnetes erstes Führungsmittel (9a) und ein an der zweiten Seite (5b) des Gestells (5) angeordnetes zweites Führungsmittel (9b), dergestalt, dass das erste (9a) und das zweite (9b) Führungsmittel ein nach innen in Richtung des knöchernen Nasengerüsts (3) ausgerichtetes erstes Führungsmittelpaar (9a, 9b) bilden;
wobei das erste (9a) und das zweite (9b) Führungsmittel zueinander parallel und im Wesentlichen parallel zur Nasenrückenlängsachse (7) ausgerichtet sind und jeweils zumindest eine erste Führungskontaktfläche (11a, 11b) bereitstellen; und
wobei die erste Führungskontaktfläche (11a, 11b) dazu eingerichtet ist, ein Operationswerkzeug (13) zu einer zumindest bereichsweisen Durchtrennung des knöchernen Nasengerüsts (3) in Richtung der Nasenrückenlängsachse (7) translatorisch zu führen; **dadurch gekennzeichnet, dass** die Vorrichtung ferner zumindest ein Fixierungsmittel (29a, 29b, 29c) umfasst,
das an der ersten oder zweiten Seite (5a, 5b) oder einem die erste und die zweite Seite (5a, 5b) verbindenden dachförmigen Abschnitt (5c) des Gestells (5) nach innen ausgerichtet angeordnet und dazu eingerichtet ist, das Gestell (5) während der Operation relativ zu dem knöchernen Nasengerüst (3) zu positionieren und daran zu fixieren.

2. Vorrichtung (1) nach Anspruch 1, weiterhin umfassend:
ein an der ersten Seite (5a) des Gestells (5) angeordnetes drittes Führungsmittel (15a) und ein an der zweiten Seite (5b) des Gestells (5) angeordnetes viertes Führungsmittel (15b), dergestalt, dass das dritte (15a) und das vierte (15b) Führungsmittel ein nach innen in Richtung des knöchernen Nasengerüsts (3) ausgerichtetes und ein zum ersten Führungsmittelpaar (9a, 9b) in der Höhe versetztes zweites Führungsmittelpaar (15a, 15b) bilden,
wobei das dritte (15a) und vierte (15b) Führungsmittel zueinander parallel und im Wesentlichen parallel zur Nasenrückenlängsachse (7) ausgerichtet sind und jeweils eine zweite Führungskontaktfläche (19a, 19b) bereitstellen; und
wobei die zweite Führungskontaktfläche (19a, 19b) dazu eingerichtet ist, das Operationswerkzeug (13) zu einer zumindest bereichsweisen Durchtrennung des knöchernen Nasengerüsts (3) in Richtung der Nasenrückenlängsachse (7) translatorisch zu führen.

3. Vorrichtung (1) nach einem der vorherigen Ansprüche, ferner umfassend:
ein an einem unteren Endbereich der ersten Seite (5a) des Gestells (5) angeordnetes fünftes Führungsmittel (21a) und ein an einem unteren Endbereich der zweiten Seite (5b) des Gestells (5) angeordnetes sechstes Führungsmittel (21b), wobei das fünfte (21a) und das sechste (21b) Führungsmittel jeweils in Richtung des knöchernen Nasengerüsts (3) ausgerichtet sind und jeweils eine dritte Führungskontaktfläche (23a, 23b) bereitstellen; und
wobei die dritte Führungskontaktfläche (23a, 23b) dazu eingerichtet ist, das Operationswerkzeug (25a, 25b) zu einer zumindest bereichsweisen Durchtrennung des knöchernen Nasengerüsts (3) im Wesentlichen längs der Nasenrückenlängsachse (7) translatorisch zu führen.

4. Vorrichtung (1) nach einem der vorherigen Ansprüche, wobei
das erste Führungsmittelpaar (9a, 9b) im Wesentlichen senkrecht zu der Nasenrückenlängsachse (7) in einer Höhe verstellbar ist; und/oder
das zweite Führungsmittelpaar (15a, 15b) im Wesentlichen senkrecht zu der Nasenrückenlängsachse (7) in einer Höhe verstellbar ist; und/oder
das fünfte (21a) und das sechste (21b) Führungsmittel im Wesentlichen senkrecht zu der Nasenrückenlängsachse (7) in einer Höhe verstellbar sind.

5. Vorrichtung (1) nach einem der vorherigen Ansprüche, ferner umfassend:
eine Kühlvorrichtung (27), die in dem oder benachbart zu dem Gestell (5) angeordnet und dazu eingerichtet ist, zumindest einen zu dem oder den Führungsmitteln benachbarten Bereich der Vorrichtung (1) zu kühlen.

6. Vorrichtung (1) nach Anspruch 1, wobei
das Gestell (5) zumindest drei Fixierungsmittel (29a, 29b, 29c) umfasst, dergestalt, dass jeweils ein Fixierungsmittel (29a, 29b, 29c) an der ersten und zweiten Seite (5a, 5b) des Gestells (5) und ein Fixierungsmittel (29a, 29b, 29c) an dem dachförmigen Abschnitt (5c) angeordnet sind.

7. Vorrichtung (1) nach einem der Ansprüche 1 bis 6, wobei
das oder die Fixierungsmittel (29a, 29b, 29c) jeweils Schrauben oder Stifte zur Fixierung der Vorrichtung (1) am knöchernen Nasengerüst (3) umfassen.

8. Vorrichtung (1) nach einem der vorherigen Ansprüche, wobei
das Gestell (5) weiterhin eine glatte und/oder beschichtete und/oder keimabweisende Oberfläche umfasst.

9. Vorrichtung (1) nach einem der vorherigen Ansprüche, wobei
eine stirnseitig ausgerichtete Kante des Gestells (5) abgerundet ist.

10. Vorrichtung (1) nach einem der vorherigen Ansprüche, wobei
die Führungsmittel jeweils als Aussparung an einer Innenseite der ersten und/oder der zweiten Seite (5a, 5b) des Gestells (5) ausgeführt sind.

11. System für eine Operation eines knöchernen Nasengerüsts (3), umfassend:
die Vorrichtung (1) nach einem der Ansprüche 1 bis 10; und
das Operationswerkzeug (13, 25a, 25b) zu einer zumindest bereichsweisen Durchtrennung des knöchernen Nasengerüsts (3) im Wesentlichen längs der Nasenrückenlängsachse (7).

12. System nach Anspruch 11, wobei
das Operationswerkzeug (13, 25a, 25b) ein Meißel, eine oszillierende Säge oder ein Piezohandstück ist.

13. System nach Anspruch 11 oder 12, wobei
die Führungskontaktflächen der Führungsmittel derart ausgestaltet sind, dass sie zumindest eine Seite des Operationswerkzeugs (13, 25a, 25b) in Richtung der Nasenrückenlängsachse (7) umgeben und das Operationswerkzeug (13, 25a, 25b) während der Operation translatorisch führen.

14. System nach einem der Ansprüche 11 bis 13, ferner umfassend:
eine Absaugvorrichtung, die an der Vorrichtung (1) oder an dem Operationswerkzeug (13, 25a, 25b) angeordnet ist und dazu eingerichtet ist, ein Kühlmedium während der Operation aus einer Umgebung des Gestells (5) und des knöchernen Nasengerüsts (3) abzusaugen.

## Claims

1. Device (1) for operation of an osseous nasal support (3), comprising:
a frame (5) having a substantially inverted U-shaped shape, aligned during the operation along a nasal bridge longitudinal axis (7), and thereby surrounding at least a portion of the osseous nasal support (3);
the frame (5) having first and second sides (5a, 5b) which are opposite to each other and each arranged adjacent to a respective side of the osseous nasal support (3) during the operation;
a first guiding means (9a) arranged at the first side (5a) of the frame (5) and a second guiding means (9b) arranged at the second side (5b) of the frame (5), such that the first (9a) and second (9b) guiding means form a first guiding means pair (9a, 9b) oriented inwardly towards the osseous nasal support (3);
said first (9a) and second (9b) guiding means being aligned parallel to each other and substantially parallel to the nasal bridge longitudinal axis (7) and each providing at least a first guiding contact face (11a, 11b); and
wherein the first guiding contact face (11a, 11b) is adapted for translationally guiding an operation tool (13) for an at least partial severing of the osseous nasal support (3) in the direction of the nasal bridge longitudinal axis (7); **characterized in that** the device further comprises
at least one fixation means (29a, 29b, 29c) arranged, in an inwardly oriented manner, at the first or second side (5a, 5b) or at a roof-shaped section (5c) of the frame (5) connecting the first and second sides (5a, 5b), and adapted to position the frame (5) relative to the osseous nasal support (3) during the operation and to fix it thereto.

2. Device (1) according to claim 1, further comprising:
a third guiding means (15a) arranged at the first side (5a) of the frame (5) and a fourth guiding means (15b) arranged at the second side (5b) of the frame (5), such that the third (15a) and fourth (15b) guiding means form a second guiding means pair (15a, 15b) oriented inwardly towards the osseous nasal support (3) and offset in height from the first guiding means pair (9a, 9b),
said third (15a) and fourth (15b) guiding means being aligned parallel to each other and substantially parallel to said nasal bridge longitudinal axis (7) and each providing a second guiding contact face (19a, 19b); and
wherein the second guiding contact face (19a, 19b) is adapted to translationally guide the operation tool (13) to at least partially sever the osseous nasal support (3) in the direction of the nasal bridge longitudinal axis (7).

3. Device (1) according to any one of the preceding claims, further comprising:
a fifth guiding means (21a) arranged at a lower end portion of the first side (5a) of the frame (5) and a sixth guiding means (21b) arranged at a lower end portion of the second side (5b) of the frame (5), the fifth (21a) and sixth (21b) guiding means each being oriented towards the osseous nasal support (3) and each providing a third guiding contact face (23a, 23b); and
wherein the third guiding contact face (23a, 23b) is adapted to translationally guide the operation tool (25a, 25b) to at least partially sever the osseous nasal support (3) substantially along the nasal bridge longitudinal axis (7).

4. Device (1) according to any one of the preceding claims, wherein
the first guiding means pair (9a, 9b) is adjustable in height substantially perpendicular to the nasal bridge longitudinal axis (7); and/or
the second guiding means pair (15a, 15b) is adjustable in height substantially perpendicular to the nasal bridge longitudinal axis (7); and/or
the fifth (21a) and sixth (21b) guiding means are adjustable in height substantially perpendicular to the nasal bridge longitudinal axis (7).

5. Device (1) according to any one of the preceding claims, further comprising:
a cooling device (27) arranged in or adjacent to the frame (5) and adapted to cool at least a portion of the device (1) adjacent to the guiding means.

6. Device (1) according to claim 1, wherein
the frame (5) comprises at least three fixation means (29a, 29b, 29c), such that one fixation means (29a, 29b, 29c) each is arranged at the first and second sides (5a, 5b) of the frame (5) and one fixation means (29a, 29b, 29c) is arranged at the roof-shaped portion (5c).

7. Device (1) according to any one of claims 1 to 6, wherein
the fixation means (29a, 29b, 29c) each comprise screws or pins for fixing the device (1) to the osseous nasal support (3).

8. Device (1) according to any one of the preceding claims, wherein
the frame (5) further comprises a smooth and/or coated and/or germ-repellent surface.

9. Device (1) according to any one of the preceding claims, wherein
a forehead-aligned edge of the frame (5) is rounded.

10. Device (1) according to any one of the preceding claims, wherein
the guide means are each configured as a recess at an inner side of the first and/or the second side (5a, 5b) of the frame (5).

11. System for an operation of an osseous nasal support (3), comprising:
the device (1) according to any one of claims 1 to 10; and
the operation tool (13, 25a, 25b) for at least partially severing the osseous nasal support (3) substantially along the nasal bridge longitudinal axis (7).

12. System according to claim 11, wherein
the operation tool (13, 25a, 25b) is a chisel, an oscillating saw or a piezo handpiece.

13. System according to claim 11 or 12, wherein
the guiding contact faces of the guiding means are configured to surround at least one side of the operation tool (13, 25a, 25b) in the direction of the nasal bridge longitudinal axis (7) and to guide the operation tool (13, 25a, 25b) translationally during the operation.

14. System according to any one of claims 11 to 13, further comprising:
a suction device arranged at the device (1) or at the operation tool (13, 25a, 25b) and adapted to suck a cooling medium from a surrounding of the frame (5) and the osseous nasal support (3) during the operation.

## Revendications

1. Dispositif (1) pour une opération d'une structure osseuse (3) du nez, comprenant :
un châssis (5) ayant une forme sensiblement en U inversé, orienté le long d'un axe longitudinal (7) de l'arête nasale pendant l'opération, tout en entourant au moins une partie de la structure osseuse (3) du nez ;
le châssis (5) ayant un premier et un deuxième côté (5a, 5b) opposés l'un à l'autre et disposés chacun de manière adjacente à un côté correspondant de la structure osseuse (3) du nez pendant l'opération ;
un premier moyen de guidage (9a) disposé sur le premier côté (5a) du châssis (5) et un deuxième moyen de guidage (9b) disposé sur le deuxième côté (5b) du châssis (5), de telle sorte que le premier (9a) et le deuxième (9b) moyens de guidage forment une première paire de moyens de guidage (9a, 9b) orientés vers l'intérieur en direction de la structure osseuse (3) du nez ;
les premier (9a) et deuxième (9b) moyens de guidage étant orientés parallèlement l'un à l'autre et sensiblement parallèlement à l'axe longitudinal (7) de l'arête nasale et fournissent chacun au moins une première surface de contact de guidage (11a, 11b) ; et
la première surface de contact de guidage (11a, 11b) étant conçue de façon à guider en translation un outil chirurgical (13) en direction de l'axe longitudinal (7) de l'arête nasale pour une section au moins partielle de la structure osseuse (3) du nez :
**caractérisé en ce que** le dispositif comprend en outre au moins un moyen de fixation (29a, 29b, 29c) disposé sur le premier ou le deuxième côté (5a, 5b) ou sur une partie en forme de toit (5c) du châssis (5) reliant les premier et deuxième côtés (5a, 5b), orienté vers l'intérieur et adapté pour, pendant l'opération, positionner le châssis (5) par rapport à la structure osseuse (3) du nez et pour le fixer à celle-ci.

2. Dispositif (1) selon la revendication 1, comprenant en outre :
un troisième moyen de guidage (15a) disposé sur le premier côté (5a) du châssis (5) et un quatrième moyen de guidage (15b) disposé sur le deuxième côté (5b) du châssis (5), de telle sorte que le troisième (15a) et le quatrième (15b) moyens de guidage présentent une orientation vers l'intérieur en direction de la structure osseuse (3) du nez et une orientation vers l'extérieur par rapport à la première paire de moyens de guidage (9a, 9b),
les troisième (15a) et quatrième (15b) moyens de guidage étant orientés parallèlement l'un à l'autre et sensiblement parallèlement à l'axe longitudinal (7) de l'arête nasale et fournissant chacun une deuxième surface de contact de guidage (19a, 19b) ; et
la deuxième surface de contact de guidage (19a, 19b) étant conçue pour guider l'outil chirurgical (13) en translation dans la direction de l'axe longitudinal (7) de l'arête nasale pour une section au moins partielle de la structure osseuse (3) du nez.

3. Dispositif (1) selon l'une des revendications précédentes, comprenant en outre :
un cinquième moyen de guidage (21a) disposé sur une partie d'extrémité inférieure du premier côté (5a) du châssis (5) et un sixième moyen de guidage (21b) disposé sur une partie d'extrémité inférieure du deuxième côté (5b) du châssis (5), le cinquième (21a) et le sixième (21b) moyens de guidage étant respectivement orientés en direction de la structure osseuse (3) du nez et fournissant chacun une troisième surface de contact de guidage (23a, 23b) ; et
la troisième surface de contact de guidage (23a, 23b) est conçue pour guider en translation l'outil chirurgical (25a, 25b) pour une section au moins partielle de la structure osseuse (3) du nez essentiellement le long de l'axe longitudinal (7) de l'arête nasale.

4. Dispositif (1) selon l'une des revendications précédentes, dans lequel
la première paire de moyens de guidage (9a, 9b) est réglable en hauteur sensiblement perpendiculairement à l'axe longitudinal (7) de l'arête nasale ; et/ou
la deuxième paire de moyens de guidage (15a, 15b) est réglable en hauteur de manière sensiblement perpendiculaire à l'axe longitudinal (7) de l'arête nasale ; et/ou
le cinquième (21a) et le sixième (21b) moyens de guidage sont réglables en hauteur sensiblement perpendiculairement à l'axe longitudinal (7) de l'arête nasale.

5. Dispositif (1) selon l'une quelconque des revendications précédentes, comprenant en outre :
un dispositif de refroidissement (27) disposé dans ou au voisinage du châssis (5) et agencé pour refroidir au moins une zone du dispositif (1) adjacente au ou aux moyens de guidage.

6. Dispositif (1) selon la revendication 1, dans lequel
le châssis (5) comprend au moins trois moyens de fixation (29a, 29b, 29c), de telle sorte qu'un moyen de fixation (29a, 29b, 29c) soit disposé sur chacun des premier et deuxième côtés (5a, 5b) du châssis (5) et qu'un moyen de fixation (29a, 29b, 29c) soit agencé sur la partie en forme de toit (5c).

7. Dispositif (1) selon l'une des revendications 1 à 6, dans lequel
le ou les moyens de fixation (29a, 29b, 29c) comprennent chacun des vis ou des broches pour fixer le dispositif (1) à la structure osseuse (3) du nez.

8. Dispositif (1) selon l'une quelconque des revendications précédentes, dans lequel
le châssis (5) comprend en outre une surface lisse et/ou revêtue et/ou aseptique.

9. Dispositif (1) selon l'une des revendications précédentes, dans lequel un bord du châssis (5) orienté du côté frontal est arrondi.

10. Dispositif (1) selon l'une des revendications précédentes, dans lequel
les moyens de guidage sont réalisés chacun sous la forme d'un évidement sur une face interne du premier et/ou du deuxième côté (5a, 5b) du châssis (5).

11. Système pour une opération d'une structure osseuse (3) du nez, comprenant :
le dispositif (1) selon l'une des revendications 1 à 10 ; et
l'outil chirurgical (13, 25a, 25b) pour une section au moins par zones de la structure osseuse (3) du nez sensiblement le long de l'axe longitudinal (7) de l'arête nasale.

12. Système selon la revendication 11, dans lequel
l'outil chirurgical (13, 25a, 25b) est un burin, une scie oscillante ou une pièce à main piézoélectrique.

13. Le système selon la revendication 11 ou 12, dans lequel
les surfaces de contact de guidage des moyens de guidage sont configurées de manière à entourer au moins un côté de l'outil chirurgical (13, 25a, 25b) dans la direction de l'axe longitudinal (7) de l'arête nasale et à guider l'outil chirurgical (13, 25a, 25b) en translation pendant l'opération.

14. Système selon l'une quelconque des revendications 11 à 13, comprenant en outre :
un dispositif d'aspiration qui est disposé sur le dispositif (1) ou sur l'outil chirurgical (13, 25a, 25b) et qui est adapté pour aspirer pendant l'opération un agent de refroidissement à partir d'un environnement du châssis (5) et de la structure osseuse (3) du nez.
